(19) Europäisches Patentamt
European Patent Office
Office européen des brevets

(11) Numéro de publication : **0 097 107**
**B1**

(12) **FASCICULE DE BREVET EUROPÉEN**

(45) Date de publication du fascicule du brevet : **18.09.85**

(21) Numéro de dépôt : **83420092.5**

(22) Date de dépôt : **02.06.83**

(51) Int. Cl.⁴ : **C 07 D333/24**, C 07 C119/06, A 01 N 43/10, A 01 N 37/50

(54) **Nouveaux dérivés de l'acide phénylimino-2 acétique, leur préparation et leur utilisation comme régulateurs de la croissance des plantes.**

(30) Priorité : **11.06.82 FR 8210371**

(43) Date de publication de la demande : **28.12.83 Bulletin 83/52**

(45) Mention de la délivrance du brevet : **18.09.85 Bulletin 85/38**

(84) Etats contractants désignés : **AT BE CH DE FR GB IT LI LU NL SE**

(56) Documents cités : **EP-A- 0 048 039**

(73) Titulaire : **RHONE-POULENC AGROCHIMIE
14-20, rue Pierre Baizet
F-69009 Lyon (FR)**

(72) Inventeur : **Bulot, Jean-Paul
49, Avenue de la République
F-77380 Combs-la-Ville (FR)**
Inventeur : **Sauli, Michel
76, Boulevard Diderot
F-75012 Paris (FR)**

(74) Mandataire : **Chaumette, Michel et al
RHONE-POULENC AGROCHIMIE BP 9163-09
F-69263 Lyon Cedex 1 (FR)**

**EP 0 097 107 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux dérivés de l'acide phénylimino-2 acétique et la préparation de ces nouveaux composés.

Elle concerne de plus des compositions régulatrices de la croissance des plantes contenant comme matière active ces dérivés de l'acide phénylimino-2 acétique ainsi que l'utilisation de ces composés dans les traitements destinés à modifier la croissance des plantes.

Dans le texte de la présente demande de brevet, l'expression « régulatrices de la croissance » est prise dans son acception courante en langue française, qui correspond à « substances de croissance » de la littérature anglo-saxonne, le mot croissance se rapportant en fait à la production de matière vivante et non seulement à la modification de la hauteur des plantes. Par « régulateurs de croissance », on entendra dans ce qui suit, des produits capables de modifier la physiologie des plantes de diverses manières.

Selon les cas, il peut s'agir par exemple :

— d'une action sur les dimensions des cellules : accélération de la germination des graines, augmentation ou réduction de la croissance,

— d'une action sur la multiplication des cellules : inhibition de la germination, bouturage, mise à fruit,

— d'interactions avec les substances de croissance naturelles : maintien ou rupture de dormance, arrêt de croissance, éclaircissage chimique des fruits, prévention de la chute des fruits, induction florale, défoliation,

— de modifications du métabolisme : augmentation de la précocité, de la productivité, etc...

La demande de brevet européen n° 78 200229.9 publiée sous le n° 0 001 655 décrit, en tant que régulateurs de la croissance des plantes des dérivés de cyanobenzyl(idène) aniline de formule générale :

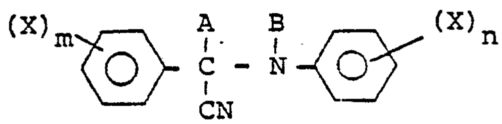

dans laquelle A et B soit représentent chacun un atome d'hydrogène, soit forment ensemble une deuxième liaison covalente. Elle indique que ces composés peuvent être utilisés pour améliorer le rendement de cultures telles que soja et haricot.

La demande de brevet européen n° 81 200 594.0 publiée sous le n° 0 048 039, décrit en tant que régulateurs de la croissance des plantes, des composés de formule générale :

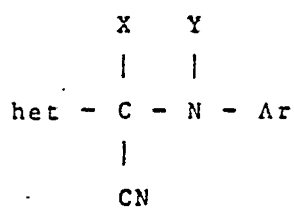

dans laquelle « het » représente un hétérocycle à 5 chaînons, éventuellement substitué, Ar représente un radical phényle éventuellement substitué et X et Y représentent ensemble une deuxième liaison, ou encore X représente un atome d'hydrogène ou un radical alkyle et Y représente un atome d'hydrogène ou un radical acyle. Elle indique que ces composés peuvent être utilisés pour améliorer le rendement de cultures telles que le soja.

On observera que tous les composés régulateurs de la croissance des plantes décrits par ces deux demandes de brevet comportent nécessairement le substituant —CN sur l'atome de carbone lié à l'atome d'azote du groupe imino (ou amino) ce qui suggère implicitement que la présence de ce substituant favorise une activité de régulateur de la croissance des plantes. Tous ces composés sont généralement préparés avec utilisation comme réactifs, de cyanures de métaux alcalins ce qui présente de sérieux inconvénients, dans le cas d'une fabrication industrielle du fait des problèmes de traitement des effluents qui en résultent.

Un but de la présente invention est de proposer des nouveaux composés régulateurs de la croissance des plantes. Un autre but de l'invention est de proposer des composés régulateurs de la croissance des plantes dont la fabrication industrielle ne présente pas les inconvénients mentionnés plus haut en ce qui concerne les produits de l'art antérieur.

Un autre but de l'invention est de proposer des régulateurs de la croissance des plantes utilisables pour améliorer le rendement des cultures, notamment du soja.

Un autre but de l'invention est de proposer des régulateurs de la croissance des plantes utilisables comme défoliants, tout particulièrement pour favoriser les récoltes dans le cas de grandes cultures en

éliminant les feuilles sans détériorer la plante elle-même.

D'autres buts pourront apparaître à l'homme du métier à la lecture de la présente demande de brevet.

Il a maintenant été trouvé que ces divers buts pouvaient être atteints au moyen des nouveaux composés décrits ci-après.

La présente invention concerne, en premier lieu, de nouveaux dérivés de l'acide phénylimino-2 acétique, caractérisés en ce qu'ils répondent à la formule générale (I) :

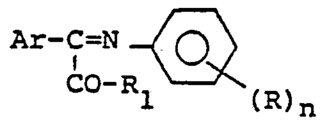

(I)

dans laquelle :

— Ar représente un radical phényle éventuellement substitué (par exemple par un ou plusieurs atomes d'halogènes ou radicaux méthyle), ou un radical hétérocyclique à cinq chaînons, contenant comme hétéroatome un atome de soufre ou d'azote, ce radical hétérocyclique étant lui-même éventuellement substitué (par exemple par un ou plusieurs atomes d'halogènes),

— R représente un substituant choisi parmi les atomes d'halogènes, les radicaux alkyle inférieurs les radicaux alkoxy inférieurs, le radical nitro, le radical hydroxyle, les radicaux halogénoalkyles inférieurs contenant de un à plusieurs atomes d'halogène (par exemple radical chlorométhyle, le radical trifluorométhyle etc),

— n est un nombre entier égal à 0, 1, 2, 3, ou 4 étant entendu que lorsque n est supérieur à 1, les substituants R peuvent être identiques ou différents,

— $R_1$ représente un radical alkoxy inférieur, amino, alkylamino inférieur ou dialkylamino dans lequel les deux parties alkyle, identiques ou différentes, comportent chacune de 1 à 6 atomes de carbone.

Au sens du présent texte, l'adjectif « inférieur » lorsqu'il qualifie un radical signifie que ce radical contient au plus six atomes de carbone.

On observera que les composés selon l'invention ne comportent pas le substituant —CN sur l'atome de carbone lié à l'atome d'azote du groupe imino. Comme on le verra plus loin, ils possèdent néanmoins d'excellentes propriétés de régulateurs de la croissance des plantes.

Parmi les composés selon la formule (I) ci-dessus, une famille préférée du fait de ses propriétés de régulateurs de la croissance des plantes comprend les composés selon la formule (I) pour lesquels :

— Ar représente le radical phényle ou un radical thiényle

— R représente un atome d'halogène (de préférence chlore ou fluor ou brome) ou un radical alkyle comportant de 1 à 4 atomes de carbone (de préférence méthyle ou éthyle, ou le radical trifluorométhyle), ou un radical alkoxy comportant de 1 à 4 atomes de carbone,

— n est égal à 1, 2 ou 3, étant entendu que lorsque n est supérieur à 1, les substituants R peuvent être identiques ou différents,

— $R_1$ représente un radical alkoxy contenant de 1 à 3 atomes de carbone, ou le radical amino ou le radical méthylamino ou le radical diméthylamino.

De plus, il a été observé que les composés répondant à la formule (I bis) ci-après :

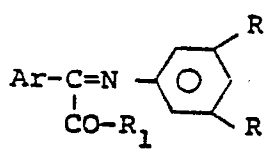

(I bis)

dans laquelle Ar et $R_1$ ont même signification que dans la formule I et R représente un atome d'halogène (de préférence le chlore) ou le radical méthyle présentaient souvent de remarquables propriétés de défoliants et pouvaient donc être utilisés pour favoriser la chute des feuilles, par exemple pour faciliter ainsi les récoltes dans le cas de grandes cultures.

La présente invention concerne la préparation des composés répondant à la formule (I) par condensation de l'aniline de formule (II) :

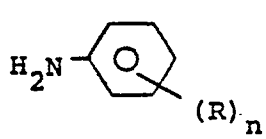

(II)

dans laquelle R et n ont la même signification que dans la formule (I) avec le dérivé de l'acide arylglyoxylique répondant à la formule (III) :

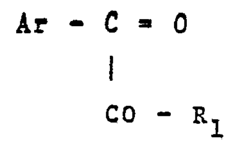

(III)

dans laquelle Ar et $R_1$ ont la même signification que dans la formule (I).

La réaction s'effectue généralement dans un solvant organique inerte, c'est-à-dire ne réagissant pas vis-à-vis des réactifs en présence, en opérant à une température comprise entre 20 °C et 150 °C environ, avantageusement à la température d'ébullition du solvant utilisé.

Comme solvants utilisables, on peut mentionner des hydrocarbures aromatiques ou aliphatiques ou cycloaliphatiques, halogénés ou non, tels que, par exemple le toluène, les xylènes, le chlorobenzène, les dichlorobenzènes ; ou des nitriles, tel que, par exemple l'acétonitrile, ou des amides, tel que par exemple le N,N diméthylformamide.

Avantageusement la réaction du composé (II) avec le composé (III) s'effectue en présence d'un agent de deshydratation usuel, tel que un acide fort (par exemple l'acide sulfurique ou l'acide p-toluène sulfonique) ou un anhydride d'acide ou un chlorure d'acide (par exemple $P_2O_5$ ou $POCl_3$).

En fin de réaction, le composé formé peut être isolé du milieu réactionnel selon les méthodes usuelles, par exemple par distillation du solvant, ou par cristallisation du composé dans le milieu réactionnel ou par filtration etc..., puis, si nécessaire, il est ensuite purifié par exemple par recristallisation dans un solvant approprié.

Les exemples ci-après décrits à titre non limitatif illustrent la préparation des composés selon l'invention ainsi que leur utilisation comme régulateurs de la croissance des plantes. Tous ces composés ont été identifiés par spectrographie infra-rouge.

## Exemple 1

Préparation du [(chloro-4-phényl) imino]-2 (thiényl-2)-2 acétate d'éthyle (composé n° 1), de formule :

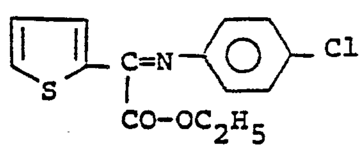

(composé n° 1)

184 g de thiényl-2 glyoxylate d'éthyle, 127,6 g de chloro-4 aniline et 1 g d'acide toluènesulfonique sont dissous dans 400 ml de toluène. La solution est chauffée à 114 °C pendant 4 heures et l'eau fournie est recueillie dans un récepteur Dean-Stark. Après concentration sous pression réduite du mélange réactionnel, le résidu huileux obtenu est dissous dans 3 litres de pentane. Cette solution est ensuite lavée avec 3 × 200 ml d'acide chlorhydrique N ; 3 × 100 ml d'eau, 3 × 200 ml de soude, N ; 3 × 200 ml d'eau puis concentrée sous pression réduite (50 °C/lmbar). Le produit brut est recristallisé dans de l'éthanol à 95 %. On obtient 180 g (rendement = 61 %) de (chloro-4 phényl)imino-2(thiényl-2)-2 acétate d'éthyle, sous forme de cristaux jaunes fondant à 48 °C.

## Exemple 2

Préparation du [(chloro-4 phényl) imino]-2(thiényl-2)-2 acétamide (composé n° 2), de formule :

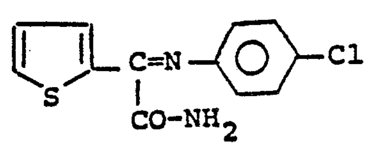

(composé n° 2)

7,7 g de thiényl-2 glyoxylamide, 6,4 g de chloro-4 aniline et 1g d'acide p-toluènesulfonique sont dissous dans 100 ml de toluène. La solution est chauffée à reflux pendant deux heures et l'eau formée est recueillie dans un récepteur de Dean Stark. Après filtration du mélange réactionnel, le solide est lavé successivement par de l'eau puis par une solution aqueuse à 10 % de bicarbonate de sodium et enfin par de l'eau. Le produit brut est ensuite cristallisé dans de l'éthanol à 95 % et on obtient ainsi 5 g de (chloro-4phényl)imino -2(thiényl-2)-2 acétamide fondant à 189 °C. Le thiényl-2 glyoxylamide a été obtenu à partir du thiényl-2 glyoxylate d'éthyle selon la méthode décrite par BRADLEY dans Ber 19 2119 (1886).

Exemple 3

En opérant selon l'une ou l'autre des méthodes décrites dans les exemples précédents, les composés ci-après ont été préparés :

[(dichloro-3,5 phényl)imino]-2 (thiényl-2)-2 acétate d'éthyle (composé n° 3)

phénylimino-2 (thiényl-2)-2 acétate d'éthyle (composé n° 4) [(chloro-3 phényl)imino]-2 (thiényl-2)-2 acétate d'éthyle (composé n° 5)

[(fluoro-4 phényl)imino]-2 (thiényl-2)-2 acétate d'éthyle (composé n° 6)

[(méthyl-4 phényl)imino]-2 (thiényl-2)-2 acétate d'éthyle (composé n° 7).

[(méthoxy-2 phényl)imino]-2 (thiényl-2)-2 acétate d'éthyle (composé n° 8)

[(chloro-4 phényl)imino]-2 (thiényl-2)-2 acétate de méthyle (composé n° 9)

[(chloro-4 phényl)imino]-2 (thiényl-2)-2 N-méthyl acétamide (composé n° 10)

[(dichloro-2,4 phényl)imino]-2 (thiényl-2)-2 acétate d'éthyle (composé n° 11)

[(dichloro-3,4 phényl)imino]-2 (thiényl-2)-2 acétate d'éthyle (composé n° 12)

[(chloro-4 méthyl-2 phényl)imino]-2 (thiényl-2)-2 acétate d'éthyle (composé n° 13)

[(chloro-2 méthyl-4 phényl)imino]-2 (thiényl-2)-2 acétate d'éthyle (composé n° 14)

[(chloro-3 méthyl-4 phényl)imino]-2 (thiényl-2)-2 acétate d'éthyle (composé n° 15)

[(méthoxy-4 phényl)imino]-2 (thiényl-2)-2 acétate d'éthyle (composé n° 16)

[(bromo-4 méthyl-2 phényl)imino]-2 (thiényl-2)-2 acétate d'éthyle (composé n° 17)

[(n-butylphényl)imino]-2 (thiényl-2)-2 acétate d'éthyle (composé n° 18)

[(diméthyl-3,5 phényl)imino]-2 (thiényl-2)-2 acétate d'éthyle (composé n° 19)

[(chloro-4 trifluorométhyl-2 phényl)imino]-2 (thiényl-2)-2 acétate d'éthyle (composé n° 20)

[(chloro-4phényl)imino]-2 phényl-2 acétate d'éthyle (composé n° 21)

[(bromo-4 phényl)imino]-2 phényl-2 acétate d'éthyle (composé n° 22)

[(méthoxy-4 phényl)imino]-2 phényl-2 acétate d'éthyle (composé n° 23)

[(iodo-4 phényl)imino]-2 phényl-2 acétate d'éthyle (composé n° 24)

[(méthyl-4 phényl)imino]-2 phényl-2 acétate d'éthyle (composé n° 25)

[(fluoro-4 phényl)imino]-2 phényl-2 acétate d'éthyle (composé n° 26)

[(dichloro-2,4 phényl)imino]-2 phényl-2 acétate d'éthyle (composé n° 27)

[(bromo-4 méthyl-2 phényl)imino]-2 phényl-2 acétate d'éthyle (composé n° 28)

[(chloro-3 méthyl-4 phényl)imino]-2 acétate d'éthyle (composé n° 29)

[(dichloro-3,4 phényl)imino]-2 phényl-2 acétate d'éthyle (composé n° 30)

[(dichloro-3,5 phényl)imino]-2 phényl-2 acétate d'éthyle (composé n° 31)

[(dichloro-3,5 phényl)imino]-2 phényl-2 acétate de méthyle (composé n° 32)

Les formules et points de fusion de ces composés 3-32 ainsi que les rendements obtenus dans leur préparation sont indiqués dans les tableaux I et II à la fin de la description.

Exemple 4

Test régulateur de croissance en serre : mise à fruit et à fleurs du haricot avec observation du résultat 4 semaines après le traitement

On prépare une solution de 25 parties en poids de la matière à tester, dans 65 parties en poids d'un mélange, en parties égales, de toluène et d'acétophénone et on ajoute 5 parties de Tween 80 (condensat d'oxyde d'éthylène et de monooléate de sorbitan) et 5 parties en poids d'un dérivé oxyéthylé d'alkylphénol. Cette solution est ensuite émulsionnée dans de l'eau distillée de façon à obtenir la concentration désirée.

La plante utilisée est le haricot nain de variété Contender, cultivé en serre, dans des pots remplis d'un mélange de terre végétale et sable, à raison de un plant de haricot par pot. Les plants sont conservés en serre climatisée, dans les conditions suivantes :

— température diurne : 23 ± 2 °C ;
— température nocturne : 18 ± 2 °C,
— éclairage : 16 heures/jour — 12 000 Lux au niveau de la plante.

Les plants sont arrosés une fois par semaine.

Lorsque les plants de haricots sont au stade « développement de la première feuille vraie », on leur applique par pulvérisation jusqu'à ruissellement la suspension aqueuse contenant la matière active à tester en utilisant 7 plants pour chaque dose. Des plants témoins sont traités, dans les mêmes conditions, par une solution de Tween 80 dans l'eau ne contenant pas de matière active, et on utilise pour cela 7 plants témoins.

Trois semaines après le traitement, on compte le nombre de fleurs et de fruits formés d'une part sur la tige principale et d'autre part sur les tiges secondaires, pour chaque plant de haricot. On compte de même le nombre de fleurs et de fruits formés sur les témoins non traités. Les résultats (moyenne rapportée à un plant de haricot) sont indiqués dans les tableaux : A, B, C, figurant à la fin de la description, chacun de ces tableaux correspondant à des essais effectués simultanément dont les résultats sont comparatifs entre eux. Les tableaux D, E, F, G, I illustrent des essais effectués selon la

même méthode que précédemment, en effectuant toutefois le relevé des résultats quatre semaines après le traitement (au lieu de trois semaines dans les essais A, B et C).

Dans ce même essai, il a été observé que, à des doses de 0,25 g/l à 3 g/l, les composés n° 3, 19, 31 et 32 présentaient une très nette activité défoliante. Les résultats observés dans cet essai sont consignés dans le tableau K.

Exemple 5

Essai de plein champ sur soja :

Dans un essai effectué en plein champ sur soja de variété « Williams », il a été observé que le composé n° 1, lorsqu'appliqué sur la culture à des doses de 0,560 kg/ha, entraînait une augmentation significative du nombre de gousses formées, en prenant comme comparaison des plants de soja non traités.

La composition utilisée pour cet essai était sous la forme d'un concentré émulsionnable comprenant respectivement pour 1 litre :

| | |
|---|---|
| — composé n° 1 | 400 g |
| — agent émulsionnant non ionique (tristyryl-phénol polyéthoxyle) | 55 g |
| — agent tensioactif anionique (alkyl arylsulfonate alcalin) | 45 g |
| — cyclohexane | 150 g |
| — xylène q.s.p. | 1 litre |

Ces résultats montrent bien les propriétés remarquables des composés selon l'invention qui peuvent être utilisés sur toutes sortes de plantes monocotylédones (notamment graminées) et dicotylédones comme celles de grandes cultures, cultures industrielles, cultures fruitières et légumières, plantes médicinales et à parfum principalement en vue d'en augmenter le rendement, ou encore, dans le cas des composés selon la formule (I bis), pour favoriser la chute des feuilles.

Comme composés préférés, du fait de leur activité biologique on peut mentionner tout particulièrement les composés 5, 6, 7, 13, 14, 15, 21 et surtout les composés 1, 2, 3, 12, 19, 31 et 32.

Pour leur emploi en pratique, les composés décrits dans la présente demande de brevet sont généralement utilisés dans des compositions qui comprennent en général, en plus de la matière active, un support inerte et/ou un agent tensioactif compatible avec la matière active et utilisable en agriculture ou horticulture. Ces compositions sont comprises dans le cadre de l'invention.

De façon générale, elles contiennent de 0,001 à 95 % en poids de matière active. Leur teneur en agent tensioactif est généralement comprise entre 0 et 20 % en poids.

Le terme « support » au sens de la présente description désigne une matière, organique ou minérale, naturelle ou synthétique, avec laquelle la matière active est associée pour faciliter son application sur la plante, sur des graines ou sur le sol, ou son transport, ou sa manipulation. Le support peut être solide (par exemple argiles) ou liquide (par exemple eau, alcools, cétones, éthers-oxydes, esters, hydrocarbures aromatiques, hydrocarbures halogénés, fractions de pétrole).

Lorsque le support est l'eau ou un solvant organique usuel, la composition comprend généralement, en plus de la matière active, un composé tel que, par exemple un agent tensioactif ou une autre substance régulatrice de la croissance des plantes.

L'agent tensioactif (ou agent de surface), utilisable dans les compositions selon l'invention, peut être un agent mouillant, dispersant ou émulsifiant, pouvant être ionique ou non ionique. On peut citer, par exemple, des non ioniques, comme les condensats d'oxyde d'éthylène avec les alcools ou avec les acides gras ou avec les alkylphénols, les esters d'acides gras et du sorbitan, les dérivés du saccharose, des anioniques, comme les sels d'acides lignosulfoniques, d'acides alkylarylsulfoniques, d'alcoylsulfosuccinates et sulfosuccinamates, des dérivés d'aminoacides, des cationiques comme des acétates d'alcoylamines ou d'imidazoline, des amphotères comme les alcoylbétaïnes ou sulfobétaïnes.

En plus de la matière active, du support et de l'agent tensioactif, les compositions selon l'invention peuvent contenir d'autres additifs tels que des agents dispersants non tensioactifs, des peptisants, colloïdes protecteurs, des épaississants, des adhésifs augmentant la résistance à la pluie, des stabilisants, des agents conservateurs, des inhibiteurs de corrosions, des colorants, des séquestrants, des anti-mousses, anti-mottants, anti-gels, etc.

Les compositions selon l'invention peuvent être préparées sous la forme de poudres pour poudrage, de poudres mouillables, de granulés autodispersibles, de solutions ou concentrés émulsionnables, d'émulsions et de dispersions.

Les poudres pour poudrage contiennent habituellement de 0,1 % à 5 % en poids de matière active, un support inerte qui peut être un support d'imprégnation tel qu'une silice et, lorsque nécessaire, de 0 à 10 % en poids de un ou plusieurs stabilisants et/ou d'autres additifs tels que des agents de pénétration, des adhésifs, des agents antimottants ou des colorants. Elles sont habituellement préparées par prémélange de la matière active avec le support inerte et le mélange est ensuite broyé dans un broyeur.

A titre d'exemple, voici la composition d'une poudre pour poudrage, selon l'invention ; les pourcentages étant exprimés en poids :

— matière active (composé 1) 1 %
— silice antimottante 10 %
— talc 89 %

Les poudres mouillables (ou poudre à pulvériser) sont habituellement préparées de manière qu'elles contiennent 20 à 95 % de matières actives, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou d'autres additifs, comme des agents de pénétration, des adhésifs, ou des agents antimottants, colorants, etc.

Les granulés et micro-granulés, généralement à faible titre de matière active, peuvent être préfabriqués puis imprégnés ou fabriqués dans la masse par exemple par atomisation ou par extrusion. Dans ce dernier cas, ils nécessitent l'adjonction de liants et de produits tensio-actifs pour assurer leur délitage rapide au sol. Ils contiennent, en général, de 0,5 à 10 % en poids de matière active, de 0 à 10 % en poids d'additifs comme des stabilisants, des agents de modification à libération lente, des liants et des solvants.

Les solutions ou concentrés émulsionnables contiennent la matière active dissout dans un solvant (généralement un hydrocarbure aromatique) et en plus, quand c'est nécessaire, un solvant auxiliaire ou cosolvant pouvant être une cétone, un ester, un éther-oxyde, etc.

En général, ils contiennent de 5 à 60 % en poids/volume de matières actives, de 2 à 20 % en poids/volume d'agent émulsifiant et peuvent être préparés, par exemple, par dissolution de matière active, dans le solvant ou dans le mélange de solvants et émulsifiant, dans une cuve équipée d'une bonne agitation et d'une circulation de fluide pour le chauffage ou le refroidissement.

A titre d'exemple, voici la composition d'une solution émulsionnable selon l'invention :

— matière active (composé 1) 400 g
— émulsifiants non ioniques/anioniques 100 g
— cétone cyclique 150 g
— solvant aromatique q.s.p. 1 litre

Voici la composition d'une autre solution émulsionnable selon l'invention :

— matière active (composé 1) 300 g
— émulsifiants non ioniques/anioniques 100 g
— méthyl-phényl-éther q.s.p. 1 litre

Tableau A

| Composé n° | Concentration g/l | Nombre de fleurs + fruits (7plants) | % par rapport au témoin |
|---|---|---|---|
| 1 | 1 | 71 | 142 |
| 2 | 1<br>3 | 61<br>58 | 122<br>116 |
| 4 | 3 | 52 | 104 |
| 5 | 3 | 81 | 162 |
| 6 | 1<br>3 | 53<br>60 | 106<br>120 |
| 7 | 1<br>3 | 65<br>67 | 130<br>134 |
| Témoin | | 50 | 100 |

Tableau B

| Composé n° | Concentration g/l | Nombre de fleurs + fruits (7 plants) | % par rapport au témoin |
|---|---|---|---|
| 11 | 1<br>3 | 55<br>52 | 115<br>108 |
| 12 | 1 | 71 | 148 |
| 13 | 1<br>0,5 | 59<br>49 | 123<br>102 |
| 14 | 1<br>3 | 49<br>66 | 102<br>137 |
| 15 | 3 | 65 | 135 |
| Témoin | | 48 | 100 |

Tableau C

| Composé n° | Concentration g/l | Nombre de fleurs + fruits (7 plants) | % par rapport au témoin |
|---|---|---|---|
| 21<br>22 | 1<br>0,5 | 81<br>66 | 147<br>120 |
| | 1<br>0,5 | 64<br>75 | 116<br>136 |
| Témoin | | 55 | 100 |

Tableau D

| Composé n° | Concentration g/l | Nombre de fleurs + fruits (7 plants | % par rapport au témoin |
|---|---|---|---|
| 16 | 2 | 94 | 145 |
| témoin | | 65 | 100 |

Tableau E

| Composé n° | Concentration g/l | Nombre de fleurs + fruits (7 plants) | % par rapport au témoin |
|---|---|---|---|
| 17 | 0,1 | 50 | 125 |
| 17 | 0,3 | 63 | 157 |
| témoin | | 40 | 100 |

Tableau F

| Composé n° | Concentration g/l | Nombre de fleurs + fruits (7 plants) | % par rapport au témoin |
|---|---|---|---|
| 10 | 1 | 122 | 127 |
| témoin | | 96 | 100 |

Tableau G

| Composé n° | Concentration g/l | Nombre de fleurs + fruits (7 plants) | % par rapport au témoin |
|---|---|---|---|
| 23. | 2 | 84 | 125 |
| témoin | | 67 | 100 |

Tableau H

| Composé n° | Concentration g/l | Nombre de fleurs + fruits (7 plants) | % par rapport au témoin |
|---|---|---|---|
| 25 | 2 | 90 | 132 |
| 26 | 2 | 90 | 132 |
| témoin | | 68 | 100 |

Tableau I

| Composé n° | Concentration g/l | Nombre de fleurs + fruits (7 plants) | % par rapport au témoin |
|---|---|---|---|
| 27 | 2 | 76 | 141 |
| 29 | 2 | 93 | 172 |
| 30 | 2 | 98 | 181 |
| témoin | | 54 | 100 |

Tableau K

| Composé n° | Concentration g/l | % de feuilles tombées après : | | |
|---|---|---|---|---|
| | | 3 jours | 5 jours | 8 jours |
| 3 | 0,25 | 80 | 100 | 100 |
| | 0,5 | 90 | 100 | 100 |
| | 2 | 80 | 100 | 100 |
| 19 | 2 | 10 | 20 | 40 |
| 31 | 0,5 | 20 | 25 | 40 |
| | 2 | 50 | 100 | 100 |
| 32 | 0,5 | 30 | 60 | 80 |
| | 2 | 40 | 100 | 100 |

Tableau l

Composés répondant à la formule ci-après

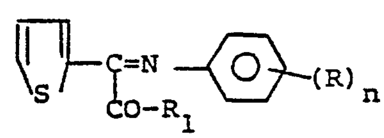

| Composé n° | 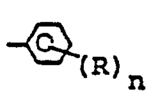 | $R_1$ | Point de fusion (°C) | Rendement % |
|---|---|---|---|---|
| 3 | 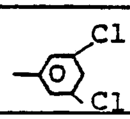 | $-OC_2H_5$ | 46 | 53 |

(Suite)

| Composé n° | 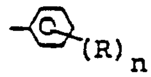 | $R_1$ | Point de fusion (°C) | Rendement % |
|---|---|---|---|---|
| 4 | 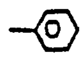 | $-OC_2H_5$ | 58 | 31 |
| 5 | 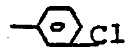 | $-OC_2H_5$ | <40 | 45 |
| 6 | 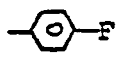 | $-OC_2H_5$ | 48 | 29 |
| 7 | 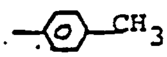 | $-OC_2H_5$ | 46 | 33 |
| 8 | 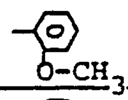 | $-OC_2H_5$ | 44 | 41 |
| 9 | 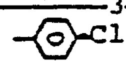 | $-OCH_3$ | 76 | 40 |
| 10 | 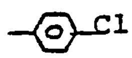 | $-NH-CH_3$ | 173 | 29 |
| 11 | 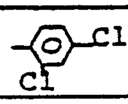 | $-OC_2H_5$ | 40 | 27 |
| 12 | 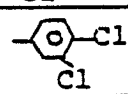 | $-OC_2H_5$ | <40 | 33 |
| 13 | 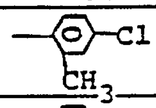 | $-OC_2H_5$ | 48 | 46 |
| 14 | 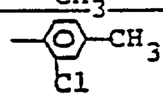 | $-OC_2H_5$ | 50 | 57 |
| 15 | 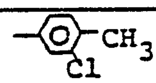 | $-OC_2H_5$ | 52 | 21 |
| 16 | 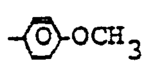 | $-OC_2H_5$ | 64 | 51 |
| 17 | 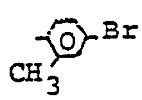 | $-OC_2H_5$ | huile | 65 |
| 18 | 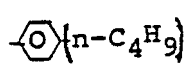 | $-OC_2H_5$ | huile | 57 |
| 19 | 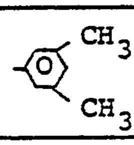 | $-OC_2H_5$ | 64 | 58 |
| 20 | 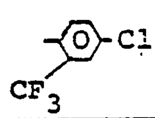 | $-OC_2H_5$ | inférieur à 50 | 47 |

Tableau II

Composés répondant à la formule ci-après

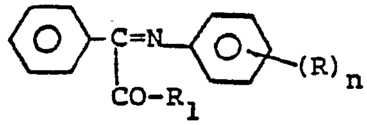

| Composé n° | 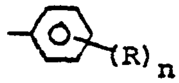 | $R_1$ | Point de fusion (°C) | Rendement % |
|---|---|---|---|---|
| 21 | 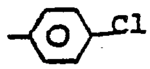 | $-OC_2H_5$ | 60 | 44 |
| 22 | 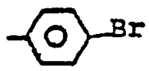 | $-OC_2H_5$ | 53 | 26 |
| 23 | 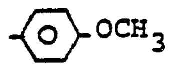 | $-OC_2H_5$ | 48 | 57 |
| 24 | 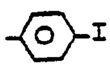 | $-OC_2H_5$ | 70 | 57 |
| 25 | 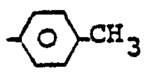 | $-OC_2H_5$ | huile | 77 |
| 26 | 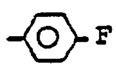 | $-OC_2H_5$ | huile | 77 |
| 27 | 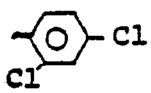 | $-OC_2H_5$ | huile | 57 |
| 28 | 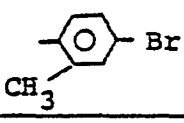 | $-OC_2H_5$ | huile | 60 |
| 29 | 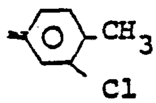 | $-OC_2H_5$ | huile | 58 |
| 30 | 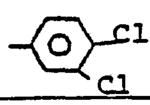 | $-OC_2H_5$ | huile | 53 |
| 31 | 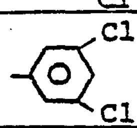 | $-OC_2H_5$ | huile | 36 |
| 32 | 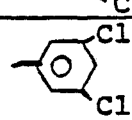 | $-OCH_3$ | 48 | 56 |

**Revendications** (pour les Etats contractants : BE, CH, DE, FR, GB, IT, LI, LU, NL, SE)

1. Dérivé de l'acide phénylimino-2 acétique caractérisé en ce qu'il répond à la formule (I)

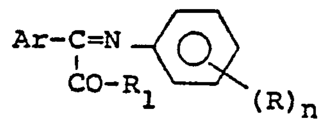

(I)

dans laquelle :

— Ar représente un radical phényle éventuelement substitué par un ou plusieurs atomes d'halogènes ou radicaux méthyle ; ou un radical hétérocyclique à cinq chaînons contenant comme hétéroatome un atome de soufre ou d'azote, lui-même éventuellement substitué par un ou plusieurs atomes d'halogènes,

— R représente un atome d'halogène ou un radical alkyle contenant de 1 à 6 atomes de carbone, ou alkoxy contenant de 1 à 6 atomes de carbone, ou nitro, ou hydroxyle ; ou halogénoalkyle contenant de 1 à 6 atomes de carbone et 1 ou plusieurs atomes d'halogènes,

— n est un entier égal à 0, 1, 2, 3, ou 4 étant entendu que lorsque n est supérieur à 1, les R peuvent être soit identiques soit différents,

— $R_1$ représente un radical alkoxy contenant de 1 à 6 atomes de carbone, amino, alkylamino contenant de 1 à 6 atomes de carbone, ou dialkylamino dans lequel chacune des parties alkyle, identiques ou différentes contient de 1 à 6 atomes de carbone.

2. Composé selon la revendication 1 caractérisé en ce qu'il répond à la formule (I) dans laquelle Ar représente le radical phényle ou un radical thiényle, R représente un atome d'halogène ou un radical alkyle comportant de 1 à 4 atomes de carbone ou le radical trifluorométhyle ou un radical alkoxy comportant de 1 à 4 atomes de carbone, n est un entier de 1 à 3, étant entendu que lorsque n est supérieur à 1 les substituants R peuvent être identiques ou différents, $R_1$ représente un radical alkoxy comportant de 1 à 3 atomes de carbone, ou amino, ou méthylamino ou diméthylamino.

3. Composé selon la revendication 1, utilisable notamment comme défoliant, caractérisé en ce qu'il répond à la formule (I bis) :

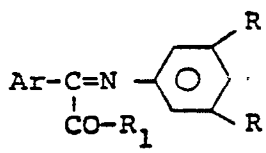

(I bis)

dans laquelle Ar, R et $R_1$ ont la même signification que dans la revendication 1.

4. Composé selon la revendication 1 caractérisé en ce que ce composé est le [(chloro-4 phényl)imino]-2 (thiényl-2)-2 acétate d'éthyle.

5. Composé selon la revendication 1 caractérisé en ce que ce composé est le [(chloro-4 phényl)imino]-2 (thiényl-2)-2 acétamide.

6. Composé selon la revendication 1 caractérisé en ce que ce composé est le [dichloro-3,5 phényl)imino]-2 (thiényl-2)-2 acétate d'éthyle.

7. Composé selon la revendication 1 caractérisé en ce que ce composé est le [diméthyl-3,5 phényl)imino]-2 (thiényl-2)-2 acétate d'éthyle.

8. Composé selon la revendication 1 caractérisé en ce que ce composé est le [dichloro-3,5 phényl)imino]-2 phényl-2 acétate d'éthyle.

9. Composé selon la revendication 1 caractérisé en ce que ce composé est le [dichloro-3,5 phényl)imino]-2 phényl-2 acétate de méthyle.

10. Composition régulatrice de la croissance des plantes caractérisée en ce qu'elle contient comme matière active un composé selon l'une des revendications 1 à 9.

11. Composition selon la revendication 10 caractérisée en ce que, en plus de la matière active, elle comprend un support inerte et/ou un agent tensioactif compatible avec la matière active et utilisable en agriculture et/ou horticulture.

12. Composition selon la revendication 11 caractérisée en ce qu'elle contient de 0,001 % à 95 % en poids de matière active.

13. Procédé de préparation d'un composé selon la revendication 1, caractérisé en ce qu'il consiste à condenser l'aniline de formule :

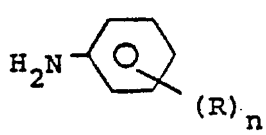

dans laquelle R et n ont la même signification que dans la revendication 1 avec le dérivé de l'acide aryl glyoxylique répondant à la formule :

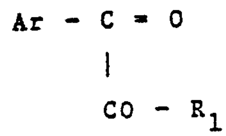

dans laquelle Ar et $R_1$ ont la même signification que dans la revendication 1.

14. Procédé pour modifier la croissance des plantes caractérisé en ce qu'il consiste à appliquer sur ces plantes une quantité efficace mais non phytotoxique vis-à-vis de ces plantes d'un composé selon l'une des revendications 1 à 9.

**Revendications** (pour l'Etat contractant AT)

1. Composition régulatrice de la croissance des plantes caractérisée en ce qu'elle contient comme matière active un dérivé de l'acide phénylimino-2 acétique répondant à la formule (I)

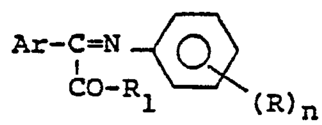

(I)

dans laquelle :

— Ar représente un radical phényle éventuellement substitué par un ou plusieurs atomes d'halogènes ou radicaux méthyle ; ou un radical hétérocyclique à cinq chaînons contenant comme hétéroatome un atome de soufre ou d'azote, lui-même éventuellement substitué par un ou plusieurs atomes d'halogènes,

— R représente un atome d'halogène ou un radical alkyle contenant de 1 à 6 atomes de carbone, ou alkoxy contenant de 1 à 6 atomes de carbone, ou nitro, ou hydroxyle ; ou halogénoalkyle contenant de 1 à 6 atomes de carbone et 1 ou plusieurs atomes d'halogènes,

— n est un entier égal à 0, 1, 2, 3, ou 4 étant entendu que lorsque n est supérieur à 1, les R peuvent être soit identiques soit différents,

— $R_1$ représente un radical alkoxy contenant de 1 à 6 atomes de carbone, amino, alkylamino contenant de 1 à 6 atomes de carbone, ou dialkylamino dans lequel chacune des parties alkyle, identiques ou différentes contient de 1 à 6 atomes de carbone.

2. Composition selon la revendication 1 caractérisée en ce qu'elle contient comme matière active un composé selon la formule (I), dans laquelle Ar représente le radical phényle ou un radical thiényle, R représente un atome d'halogène ou un radical alkyle comportant de 1 à 4 atomes de carbone ou le radical trifluorométhyle ou un radical alkoxy comportant de 1 à 4 atomes de carbone, n est un entier de 1 à 3, étant entendu que lorsque n est supérieur à 1 les substituants R peuvent être identiques ou différents et $R_1$ représente un radical alkoxy comportant de 1 à 3 atomes de carbone, ou amino, ou méthylamino ou diméthylamino.

3. Composition selon la revendication 1, utilisable notamment comme agent défoliant caractérisée en ce qu'elle contient comme matière active un composé répondant à la formule (I bis)

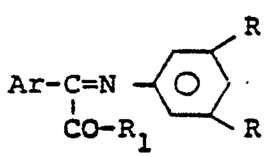

dans laquelle Ar, R et $R^1$ ont la même signification que dans la revendication 1.

4. Composition selon la revendication 1 caractérisée en ce qu'elle contient comme matière active le [(chloro-4 phényl)imino]-2 (thiényl-2)-2 acétate d'éthyle.

5. Composition selon la revendication 1 caractérisée en ce qu'elle contient comme matière active le [(chloro-4 phényl)imino]-2 (thiényl-2)-2 acétamide.

6. Composition selon la revendication 1 caractérisée en ce qu'elle contient comme matière active le [(dichloro-3,5 phényl) imino]-2 (thiényl-2)-2 acétate d'éthyle.

7. Composition selon la revendication 1 caractérisée en ce qu'elle contient comme matière active [(diméthyl-3,5 phényl)imino]-2 (thiényl-2)-2 acétate d'éthyle.

8. Composition selon la revendication 1 caractérisée en ce qu'elle contient comme matière active le [(dichloro-3,5 phényl) imino]-2 phényl-2 acétate d'éthyle.

9. Composition selon la revendication 1 caractérisée en ce qu'elle contient comme matière active le [(dichloro-3,5, phényl) imino]-2 phényl-2 acétate de méthyle.

10. Composition selon la revendication 1 caractérisée en ce que, en plus de la matière active, elle

comprend un support inerte et/ou un agent tensioactif compatible avec la matière active et utilisable en agriculture et/ou horticulture.

11. Composition selon l'une des revendications 1 à 10 caractérisé en ce qu'elle contient de 0,001 % à 95 % en poids de matière active.

12. Procédé de préparation d'un composé répondant à la formule (I)

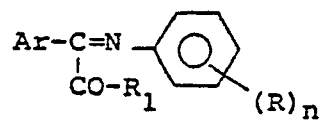

(I)

dans laquelle Ar, $R_1$, R et n ont la même signification que dans la revendication 1, caractérisé en ce qu'il consiste à condenser l'aniline de formule :

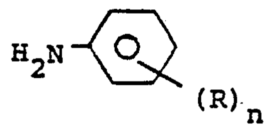

dans laquelle R et n ont la même signification que précédemment avec le dérivé de l'acide aryl glyoxylique répondant à la formule :

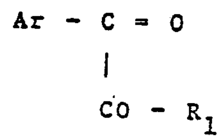

dans laquelle Ar et $R_1$ ont la même signification que précédemment.

13. Procédé pour modifier la croissance des plantes caractérisé en ce qu'il consiste à appliquer sur ces plantes une quantité efficace mais non phytotoxique vis-à-vis de ces plantes d'une composition selon l'une des revendications 1 à 11.

**Claims** (for the Contracting States : BE, CH, DE, FR, GB, IT, LI, LU, NL, SE)

1. 2-phenyliminoacetic acid derivative characterised in that it corresponds to the formula (I)

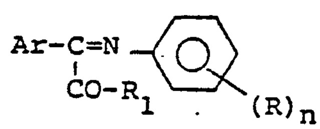

(I)

in which :

— Ar represents a phenyl radical optionally substituted by one or more halogen atoms or methyl radicals, or a heterocyclic radical with five ring members, containing a sulphur or nitrogen atom as the heteroatom, this heterocyclic radical itself being optionally substituted by one or more halogen atoms,

— R represents a halogen atom, an alkyl radical containing from 1 to 6 carbon atoms, an alkoxy radical containing from 1 to 6 carbon atoms, a nitro radical, a hydroxyl radical or a halogenoalkyl radical containing from 1 to 6 carbon atoms and 1 or more halogen atoms,

— n is an integer equal to 0, 1, 2, 3 or 4, it being understood that if n is greater than 1, the substituents R can be either identical or different, and

— $R_1$ represents an alkoxy radical containing from 1 to 6 carbon atoms, an amino radical, an alkylamino radical containing from 1 to 6 carbon atoms or a dialkylamino radical in which each of the alkyl parts, which are identical or different, contains from 1 to 6 carbon atoms.

2. Compound according to claim 1 characterised in that it corresponds to the formula (I) in which Ar represents the phenyl radical or a thienyl radical, R represents a halogen atom or an alkyl radical containing from 1 to 4 carbon atoms, the trifluoromethyl radical or an alkoxy radical containing from 1 to 4 carbon atoms, n is an integer from 1 to 3, it being understood that if n is greater than 1, the substituents R can be identical or different, and $R_1$ represents an alkoxy radical containing from 1 to 3 carbon atoms, an amino radical, a methylamino radical or a dimethylamino radical.

3. Compound according to claim 1, which can be used in particular as a defoliant, characterised in that it corresponds to the formula (I bis) :

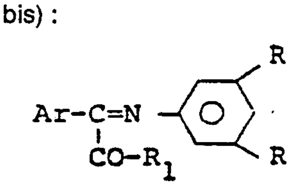

(I bis)

in which Ar, R and $R_1$ have the same meanings as in claim 1.

4. Compound according to claim 1, characterised in that this compound is ethyl 2-[(4-chlorophenyl)-imino]-2-(thien-2-yl)-acetate.

5. Compound according to claim 1, characterised in that this compound is 2-[(4-chlorophenyl)-imino]-2-(thien-2-yl)-acetamide.

6. Compound according to claim 1, characterised in that this compound is ethyl 2-[(3,5-dichlorophenyl)-imino]-2-(thien-2-yl)-acetate.

7. Compound according to claim 1, characterised in that this compound is ethyl 2-[(3,5-dimethylphenyl)-imino]-2-(thien-2-yl)-acetate.

8. Compound according to claim 1, characterised in that this compound is ethyl 2-[(3,5-dichlorophenyl)-imino]-2-phenylacetate.

9. Compound according to claim 1, characterised in that this compound is methyl 2-[(3,5-dichlorophenyl)-imino)]-2-phenylacetate.

10. Composition for regulating plant growth, characterised in that it contains a compound according to one of claims 1 to 9 as the active ingredient.

11. Composition according to claim 10, characterised in that in addition to the active ingredient, it contains an inert carrier and/or a surface-active agent which is compatible with the active ingredient and which can be used in agriculture and/or horticulture.

12. Composition according to claim 11, characterised in that it contains from 0,001 % to 95 % by weight of active ingredient.

13. Process for the preparation of a compound according to claim 1, characterised in that it consists in condensing the aniline of the formula :

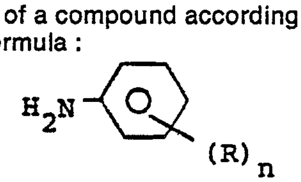

in which R and n have the same meanings as in claim 1, with the arylglyoxylic acid derivative of the formula :

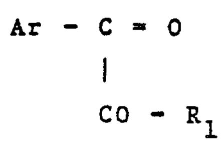

in which Ar and $R_1$ have the same meanings as in claim 1.

14. Process for modifying plant growth, characterised in that it consists in applying, to these plants, an amount of a compound according to one of claims 1 to 9, this amount being effective but non-phytotoxic towards these plants.

**Claims** (for the Contracting State AT)

1. Plant growth regulating composition characterised in that it contains as the active ingredient a 2-phenyliminoacetic acid derivative corresponding to the formula (I) :

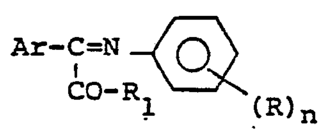

(I)

in which :

— Ar represents a phenyl radical optionally substituted by one or more halogen atoms or methyl radicals, or a heterocyclic radical with five ring members, containing a sulphur or nitrogen atom as the heteroatom, this heterocyclic radical itself being optionally substituted by one or more halogen atoms,

— R represents a halogen atom, an alkyl radical containing from 1 to 6 carbon atoms, an alkoxy radical containing from 1 to 6 carbon atoms, a nitro radical, a hydroxyl radical or a halogenoalkyl radical containing from 1 to 6 carbon atoms and 1 or more halogen atoms,

— n is an integer equal to 0, 1, 2, 3 or 4, it being understood that if n is greater than 1, the substituents R can be either identical or different, and

— $R_1$ represents an alkoxy radical containing from 1 to 6 carbon atoms, an amino radical, an alkylamino radical containing from 1 to 6 carbon atoms or a dialkylamino radical in which each of the alkyl parts, which are identical or different, contains from 1 to 6 carbon atoms.

2. Composition according to claim 1 characterised in that it contains as the active ingredient a compound corresponding to the formula (I) in which :

— Ar represents the phenyl radical or a thienyl radical, R represents a halogen atom or an alkyl radical containing from 1 to 4 carbon atoms, the trifluoromethyl radical or an alkoxy radical containing from 1 to 4 carbon atoms, n is an integer from 1 to 3, it being understood that if n is greater than 1, the substituents R can be identical or different, and $R_1$ represents an alkoxy radical containing from 1 to 3 carbon atoms, an amino radical, a methylamino radical or a dimethylamino radical.

3. Composition according to claim 2, which can be used in particular as a defoliant, characterised in that it contains as the active ingredient a compound corresponding to the formula (I bis)

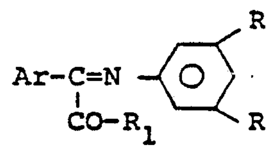

(I bis)

in which Ar, R and $R_1$ have the same meanings as in claim 1.

4. Composition according to claim 1 characterised in that it contains as active ingredient ethyl 2-[(4-chlorophenyl)-imino]-2-(thien-2-yl)-acetate.

5. Composition according to claim 1 characterised in that it contains as active ingredient 2-[(4-chlorophenyl)-imino]-2-(thien-2-yl)-acetamide.

6. Composition according to claim 1, characterised in that it contains as active ingredient ethyl 2-[(3,5-dichlorophenyl)-imino]-2-(thien-2-yl)-acetate.

7. Composition according to claim 1, characterised in that it contains as active ingredient ethyl 2-[(3,5-dimethylphenyl)-imino]-2-(thien-2-yl)-acetate.

8. Composition according to claim 1 characterised in that it contains as active ingredient ethyl 2-[(3,5-dichlorophenyl)-imino]-2-phenylacetate.

9. Composition according to claim 1 characterised in that it contains as active ingredient methyl 2-[(3,5-dichlorophenyl)-imino)]-2-phenylacetate.

10. Composition according to claim 9 characterised in that it comprises, in addition to the active ingredient, an inert carrier and/or a surface-active agent which is compatible with the active ingredient and which can be used in agriculture and/or horticulture.

11. Composition according to one of the claims 1 to 10 characterised in that it contains from 0.001 % to 95 % by weight of active ingredient.

12. A process for the preparation of a compound corresponding to the formula (I) :

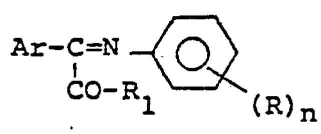

(I)

in which Ar, $R_1$, R and n have the same meaning as in claim 1, characterised in that it consists in condensing the aniline of the formula :

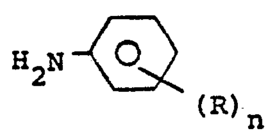

in which R and n have the same meanings as above, with the arylglyoxylic acid derivative of the formula :

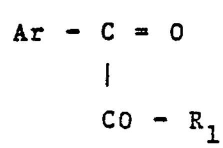

in which Ar and $R_1$ have the same meanings as above.

13. A process for modifying plant growth, characterised in that it consists in applying, to these plants, an amount of a composition according to one of claims 1 to 11, this amount being effective but non-phytotoxic towards these plants.

**Patentansprüche** (für die Vertragsstaaten : BE, CH, DE, FR, GB, IT, LI, LU, NL, SE)

1. Derivat der 2-Phenyliminoessigsäure, dadurch gekennzeichnet, daß es der Formel (I)

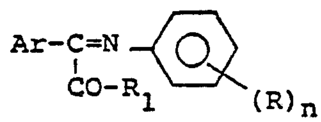

(I)

entspricht, in welcher

— Ar einen gegebenenfalls durch ein oder mehrere Halogenatome oder Methylreste substituierten Phenylrest oder einen fünfgliedrigen heterocyclischen Rest mit einem Schwefeloder Stickstoffatom als Heteroatom bedeutet, der seinerseits gegebenenfalls durch ein oder mehrere Halogenatom(e) substituiert ist,

— R für ein Halogenatom oder einen Alkylrest mit 1 bis 6 Kohlenstoffatomen oder einen Alkoxyrest mit 1 bis 6 Kohlenstoffatomen oder Nitro oder Hydroxyl oder für Halogenalkyl mit 1 bis 6 Kohlenstoffatomen und einem oder mehreren Halogenatomen steht,

— n eine ganze Zahl gleich 0, 1, 2, 3 oder 4 bedeutet, wobei bei n größer als 1 die Reste R gleich oder verschieden sein können,

— $R_1$ einen Alkoxyrest mit 1 bis 6 Kohlenstoffatomen, den Aminrest einen Alkylaminrest mit 1 bis 6 Kohlenstoffatomen oder einen Dialkylaminrest, in welchem jeder der Alkylreste, die gleich oder verschieden sind, 1 bis 6 Kohlenstoffatome enthält, darstellt.

2. Verbindung nach Anspruch 1, dadurch gekennzeichnet, daß sie der Formel (I) entspricht, in welcher Ar den Phenyl- oder einen Thienylrest, R ein Wasserstoffatom oder einen Alkylrest mit 1 bis 4 Kohlenstoffatomen oder den Trifluormethylrest oder einen Alkoxyrest mit 1 bis 4 Kohlenlenstoffatomen bedeutet, n für eine ganze Zahl von 1 bis 3 steht, wobei bei n größer als 1 die Substituenten R gleich oder verschieden sein können, und $R_1$ einen Alkoxyrest mit 1 bis 3 Kohlenstoffatomen oder den Amin-, Methylamin- oder Dimethylaminrest darstellt.

3. Verbindung nach Anspruch 1, die insbesondere als Entlaubungsmittel verwendbar ist, dadurch gekennzeichnet, daß sie der Formel (I bis)

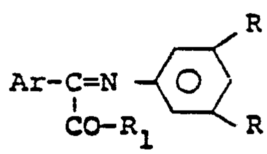

(I bis)

entspricht, in welcher Ar, R und $R_1$ die gleiche Bedeutung wie in Anspruch 1 haben.

4. Verbindung nach Anspruch 1, dadurch gekennzeichnet, daß diese Verbindung das Äthyl-2-[(4-Chlorphenyl)imino]-2-(2-thienyl) acetat ist.

5. Verbindung nach Anspruch 1, dadurch gekennzeichnet, daß diese Verbindung das 2-[(4-Chlorphenyl) imino]-2-(2-thienyl) acetamid ist.

6. Verbindung nach Anspruch 1, dadurch gekennzeichnet, daß diese Verbindung das Äthyl-2-[(3,5-Dichlorphenyl) imino]-2-(2--thienyl) acetat ist.

7. Verbindung nach Anspruch 1, dadurch gekennzeichnet, daß diese Verbindung das Äthyl-2-[3,5-Dimethylphenyl) imino]-2-(2-thienyl) acetat ist.

8. Verbindung nach Anspruch 1, dadurch gekennzeichnet, daß diese Verbindung das Äthyl-2-[(3,5-Dichlorphenyl) imino]-2-phenylacetat ist.

9. Verbindung nach Anspruch 1, dadurch gekenzeichnet, daß diese Verbindung das Methyl-2-[(3,5-Dichlorphenyl) imino]-2-phenylacetat ist.

10. Zusammensetzung zur Regulierung des Pflanzenwachstums, dadurch gekennzeichnet, daß sie als Wirkstoff eine Verbindung nach einem der Ansprüche 1 bis 9 enthält.

11. Zusammensetzung nach Anspruch 10, dadurch gekennzeichnet, daß sie zusätzlich zu dem Wirkstoff einen inerten Träger und/oder ein mit dem Wirkstoff verträgliches und in Landwirtschaft und/oder Gartenbau anwendbares Tensid enthält.

12. Zusammensetzung nach Anspruch 11, dadurch gekennzeichnet, daß sie 0,001 bis 95 Gew.-% Wirkstoff enthält.

13. Verfahren zur Herstellung einer Verbindung nach Anspruch 1, dadurch gekennzeichnet, daß es daraus besteht, das Anilin der Formel

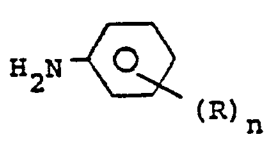

in welcher R und n die gleiche Bedeutung wie in Anspruch 1 haben, mit einem Derivat der Arylglyoxylsäure der Formel

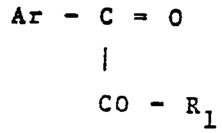

in welcher Ar und $R_1$ die gleiche Bedeutung wie in Anspruch 1 haben, umzusetzen.

14. Verfahren zur Beeinflussung des Pflanzenwachstums, dadurch gekennzeichnet, daß es daraus besteht, auf die Pflanzen eine wirksame, jedoch gegenüber diesen Pflanzen nicht phytotoxische Menge einer Verbindung nach einem der Ansprüche 1 bis 9 aufzubringen.

**Patentansprüche** (für den Vertragsstaat AT)

1. Zusammensetzung zur Regulierung des Pflanzenwachstums, dadurch gekennzeichnet, daß sie als Wirkstoff ein Derivat der 2-Phenyliminoessigsäure der Formel (I)

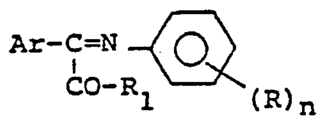

(I)

enthält, in welcher

— Ar einen gegebenenfalls durch ein oder mehrere Halogenatome oder Methylreste substituierten Phenylrest oder einen fünfgliedrigen heterocyclischen Rest mit einem Schwefel- oder Stickstoffatom als Heteroatom bedeutet, der seinerseits gegebenenfalls durch ein oder mehrere Halogenatom(e) substituiert ist,

— R für ein Halogenatom oder einen Alkylrest mit 1 bis 6 Kohlenstoffatomen oder einen Alkoxyrest mit 1 bis 6 Kohlenstoffatomen oder Nitro oder Hydroxyl oder für Halogenalkyl mit 1 bis 6 Kohlenstoffatomen und einem oder mehreren Halogenatomen steht,

— n eine ganze Zahl gleich 0, 1, 2, 3 oder 4 bedeutet, wobei bei n größer als 1 die Reste R gleich oder Verschieden sein können,

— $R_1$ einen Alkoxyrest mit 1 bis 6 Kohlenstoffatomen, den Aminrest einen Alkylaminrest mit 1 bis 6 Kohlenstoffatomen oder einen Dialkylaminrest, in welchem jeder der Alkylreste die gleich oder verschieden sind, 1 bis 6 Kohlenstoffatome enthält, darstellt.

2. Zusammensetzung nach Anspruch 1, dadurch gekennzeichnet, daß sie als Wirkstoff eine Verbindung der Formel (I) enthält, in welcher Ar den Phenyl- oder einen Thienylrest, R ein Wasserstoffatom oder einen Alkylrest mit 1 bis 4 Kohlenstoffatomen oder den Trifluormethylrest oder einen Alkoxyrest mit 1 bis 4 Kohlenstoffatomen bedeutet, n für eine ganze Zahl von 1 bis 3 steht, wobei bei n größer als 1 die Substituenten R gleich oder verschieden sein können, und $R_1$ einen Alkoxyrest mit 1 bis 3 Kohlenstoffatomen oder den Amin-, Methylamin- oder Dimethylaminrest darstellt.

3. Zusammensetzung nach Anspruch 1, die insbesonder als Entlaubungsmittel anwendbar ist, dadurch gekennzeichnet, daß sie als Wirkstoff eine Verbindung der Formel (I bis) enthält :

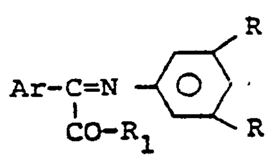

in welcher Ar, R und $R_1$ die gleiche Bedeutung wie in Anspruch 1 haben.

4. Zusammensetzung nach Anspruch 1, dadurch gekennzeichnet, daß sie als Wirkstoff das Ethyl-2-[(4-Chlorphenyl) imino]-2-(2-thienyl) acetat enthält.

5. Zusammensetzung nach Anspruch 1, dadurch gekennzeichnet, daß sie als Wirkstoff das [(4-Chlorphenyl) imino]-2-(2-thienyl) acetamid enthält.

6. Zusammensetzung nach Anspruch 1, dadurch gekennzeichnet, daß sie als Wirkstoff das Ethyl-[(3,5-Dichlorphenyl) imino]-2-(2-thienyl) acetat enthält.

7. Zusammensetzung nach Anspruch 1, dadurch gekennzeichnet, daß sie als Wirkstoff das Ethyl-[(3,5-Dimethylphenyl) imino]-2-(2-thienyl) acetat enthält.

8. Zusammensetzung nach Anspruch 1, dadurch gekennzeichnet, daß sie als Wirkstoff das Ethyl-2-[(3,5-Dichlorphenyl) imino]-2-phenylacetat enthält.

9. Zusammensetzung nach Anspruch 1, dadurch gekennzeichnet, daß sie als Wirkstoff das Methyl-[(3,5-Dichlorphenyl) imino]-2-phenylacetat enthält.

10. Zusammensetzung nach Anspruch 1, dadurch gekennzeichnet, daß sie zusätzlich zu dem Wirkstoff einen inerten Träger und/oder ein mit dem Wirkstoff verträgliches und in Landwirtschaft und/oder Gartenbau anwendbares Tensid enthält.

11. Zusammensetzung nach einem der Ansprüche 1 bis 10, dadurch gekennzeichnet, daß sie 0,001 bis 95 Gew.% Wirkstoff enthält.

12. Verfahren zur Herstellung einer Verbindung der Formel (I)

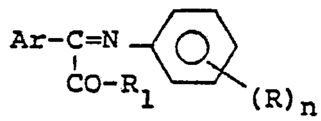

(I)

in welcher Ar, $R_1$, R und n die gleiche Bedeutung wie in Anspruch 1 haben, dadurch gekennzeichnet, daß es daraus besteht, das Anilin der Formel

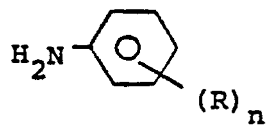

in welcher R und n die gleiche oben genannte Bedeutung haben, mit einem Derivat der Arylglyoxylsäure der Formel

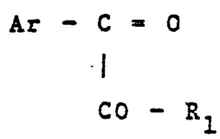

in welcher Ar und $R_1$ die gleiche oben genannte Bedeutung haben, umzusetzen.

13. Verfahren zur Beeinflussung des Pflanzenwachstums, dadurch gekennzeichnet, daß es daraus besteht, auf die Pflanzen eine wirksame, jedoch gegenüber diesen Pflanzen nicht phytotoxische Menge eine Zusammensetzung nach einem der Ansprüche 1 bis 11 aufzubringen.